# EUROPEAN PATENT APPLICATION

(11) **EP 4 464 233 A2**
(43) Date of publication of application: **20.11.2024**
(21) Application number: 23737186.9
(22) Date of filing: 09.01.2023
(51) Int. Cl.: A61B 1/00

(54) **ENDOSCOPE**

(30) Priority: 10.01.2022 CN 202220053494 U
(71) Applicant: Hangzhou Hikimaging Technology Co., Ltd., Hangzhou, Zhejiang 310051 (CN)
(72) Inventor: CHEN, Yanting, Hangzhou, Zhejiang 310051 (CN); ZHAO, Zefeng, Hangzhou, Zhejiang 310051 (CN); ZHANG, Pengfei, Hangzhou, Zhejiang 310051 (CN)
(74) Representative: Vogelbruch, Keang
(86) International application number: PCT/CN2023/071269
(87) International publication number: WO 2023/131324

(57) **Abstract**

The present application discloses an endoscope. The endoscope includes a probe tube (100) and a window assembly (200). The probe tube (100) includes a first tube body (110), which includes an opening end (111). The window assembly (200) is in blocking fit with an opening at the opening end (111). The window assembly (200) includes a sleeving section (211) disposed around the opening end (111). An inner wall surface of the sleeving section (211) serves as a first sealing surface (2111), an outer wall surface of the opening end (111) serves as a second sealing surface (1111), and the first sealing surface (2111) is in sealing fit with the second sealing surface (1111).

## Description

### TECHNICAL FIELD

The present application relates to the technical field of medical equipment, and in particular to an endoscope.

### BACKGROUND

With the progress and development of science and technology, the level of medical science and technology is constantly improving, such that endoscopes, as a kind of optical instrument, can enter the stomach through the mouth or enter the body through other natural orifices to expand the field of view of doctors and assist the doctors in observing or treating sites of lesion of patients. Therefore, the endoscopes have a wide range of applications in clinical medicine.

### SUMMARY

Embodiments of the present application aim to disclose an endoscope.

In a first aspect, the embodiments of the present application disclose an endoscope, which includes a probe tube and a window assembly. The probe tube includes a first tube body, which includes an opening end. The window assembly is in blocking fit with an opening at the opening end. The window assembly includes a sleeving section disposed around the opening end. An inner wall surface of the sleeving section serves as a first sealing surface, an outer wall surface of the opening end serves as a second sealing surface, and the first sealing surface is in sealing fit with the second sealing surface.

With the endoscope according to the embodiments of the present application, the outer wall surface of the opening end of the first tube body of the probe tube is provided as the second sealing surface, while the inner wall surface of the sleeving section of the window assembly is provided as the first sealing surface, and the sleeving section is disposed around the opening end, such that the first sealing surface can be in sealing fit with the second sealing surface. In this way, an area of the sealing fit between the window assembly and the probe tube can be increased through the sealing fit between the first sealing surface and the second sealing surface, such that the sealing performance between the window assembly and the probe tube can be improved.

### BRIEF DESCRIPTION OF DRAWINGS

FIG. 1 is a sectional structural diagram illustrating an endoscope according to embodiments of the present application at a field-of-view end.
FIG. 2 and FIG. 3 are schematic diagrams illustrating overall structures of an endoscope according to embodiments of the present application at different viewing angles.
FIG. 4 is a schematic diagram illustrating a partial structure of the endoscope shown in FIG. 3.
FIG. 5 and FIG. 6 are perspective structural diagrams illustrating a mounting cover of an endoscope according to embodiments of the present application at different angles.
FIG. 7 is a sectional structural diagram illustrating the mounting cover shown in FIG. 6.

### Description of reference numerals:

100-probe tube, 110-first tube body, 111-opening end, 1111-second sealing surface, 120-second tube body, 121-fifth sealing surface, 130-gap, 131-first sub-gap, 132-second sub-gap;
200-window assembly, 210-mounting cover, 211-sleeving section, 2111-first sealing surface, 212-cover body section, 2121-annular mounting portion, 2121a-mounting hole, 2121b-third sealing surface, 213-sixth sealing surface, 220-window, 221-fourth sealing surface.

### DETAILED DESCRIPTION

Embodiments of the present application will be clearly and completely described below in conjunction with the accompanying drawings in the embodiments of the present application. It is to be understood that the described embodiments are a part of the embodiments of the present application, and not all of the embodiments. Based on the embodiments in the present application, all other embodiments obtained by those ordinary skilled in the art without any creative efforts fall within the scope of protection of the present application.

Terms "first," "second," and the like in the description and claims of the present application are used for distinguishing between similar objects, but not for describing a particular order or sequence. It is to be understood that the terms so used are interchangeable, where appropriate, such that the embodiments of the present application can be implemented in an order other than those illustrated or described herein, and that the objects distinguished by "first," "second," and the like are generally of one kind without limiting the number of the objects, e.g., the first object may be one or more than one.

Since an endoscope is used in the human body, it is necessary to perform low-temperature plasma sterilization or high-temperature and high-pressure sterilization on the endoscope before use, which requires the endoscope to have a good air tightness. However, in a conventional endoscope, a tube assembly is directly welded to an end cover, which results in a poor air tightness at a field-of-view end of the endoscope, and is prone to cause damage to the internal structure of the endoscope during the sterilization.

In view of this, embodiments of the present application provide an endoscope. The specific type of the endoscope is not limited in the embodiments of the present application. For example, the endoscope may include a laparoscope, a nasal endoscope, an ear endoscope, an intestinal endoscope, a gastric endoscope, or the like.

The endoscope according to the embodiments of the present application will be described in detail below with specific embodiments and application scenarios thereof in conjunction with the drawings.

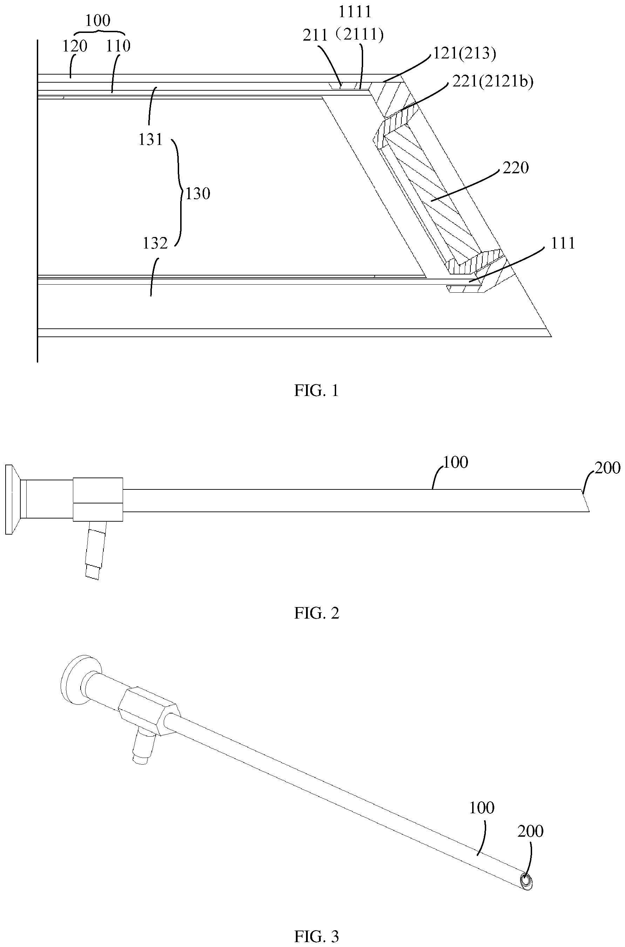

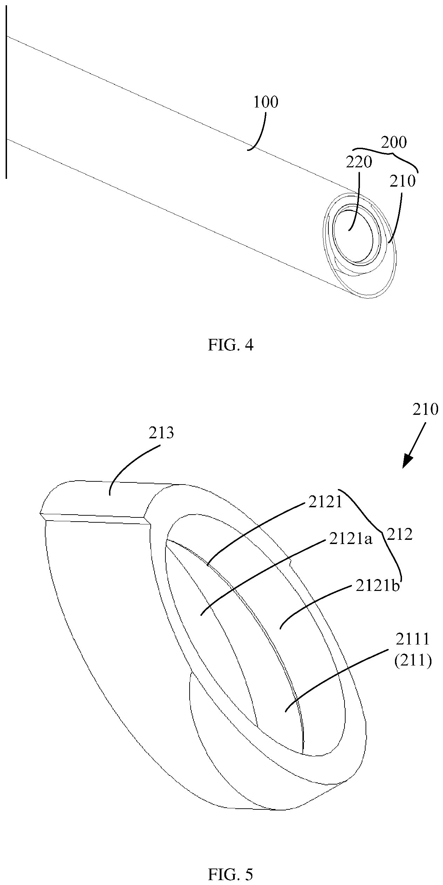

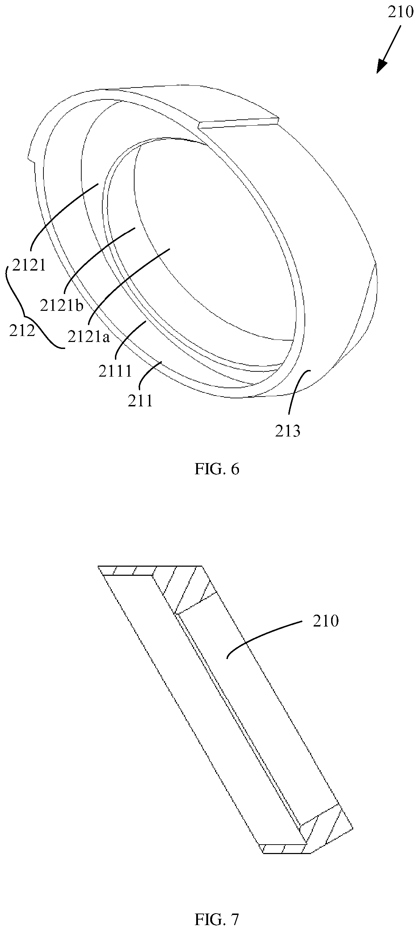

As shown in FIG. 1 to FIG. 7, embodiments of the present application disclose an endoscope, which includes a probe tube 100 and a window assembly 200.

The probe tube 100 is an insertion tube of the endoscope, which can be inserted into the human body through the mouth or other natural orifices, such that the window assembly 200 can reach a site of lesion of a patient, thereby assisting a medical staff in diagnosing or treating the site of lesion of the patient. The window assembly 200 is a component of the endoscope capable of realizing an observation function.

The probe tube 100 includes a first tube body 110, which is one of base members in the probe tube 100 for providing a mounting base. Moreover, a structure in the endoscope that can play a major functional role may be provided in the first tube body 110, such that the first tube body 110 can play a role in protecting the structure mounted therein. The first tube body 110 includes an opening end 111, and the window assembly 200 is in blocking fit with an opening at the opening end 111, such that the blocking fit between the opening at the opening end 111 and the window assembly 200 can initially form the basis for a sealing connection between the window assembly 200 and the first tube body 110.

The window assembly 200 includes a sleeving section 211, which is disposed around the opening end 111, an inner wall surface of the sleeving section 211 serves as a first sealing surface 2111, an outer wall surface of the opening end 111 serves as a second sealing surface 1111, and the first sealing surface 2111 is in sealing fit with the second sealing surface 1111. The first sealing surface 2111 and the second sealing surface 1111 are provided such that an area of the sealing fit between the window assembly 200 and the first tube body 110 is increased, thereby improving the sealing performance between the window assembly 200 and the probe tube 100, which solves the problem that the main functional structure provided inside the endoscope is prone to be damaged during the sterilization of the endoscope due to the poor sealing performance at a field-of-view end of the endoscope.

It should be noted that the sealing fit according to the present application may be realized by welding, adhesion, or direct bonding, which is not particularly limited in the present application.

With the endoscope according to the embodiments of the present application, the outer wall surface of the opening end 111 of the first tube body 110 of the probe tube 100 is provided as the second sealing surface 1111, while the inner wall surface of the sleeving section 211 of the window assembly 200 is provided as the first sealing surface 2111, and the sleeving section 211 is disposed around the opening end 111, such that the first sealing surface 2111 can be in sealing fit with the second sealing surface 1111. In this way, an area of the sealing fit between the window assembly 200 and the probe tube 100 can be increased through the sealing fit between the first sealing surface 2111 and the second sealing surface 1111, such that the sealing performance between the window assembly 200 and the probe tube 100 can be improved.

In the endoscope according to the embodiments of the present application, the window assembly 200 may include a mounting cover 210 and a window 220. The mounting cover 210 is a mounting base for the window 220, and the sleeving section 211 described above may be included in the mounting cover 210. The mounting cover 210 may be provided with a mounting hole 2121a, such that at least a portion of the window 220 can be secured in the mounting hole 2121a. An inner wall surface of the mounting hole 2121a serves as a third sealing surface 2121b, a surface of the window 220 facing the third sealing surface 2121b serves as a fourth sealing surface 221, and the third sealing surface 2121b is in sealing fit with the fourth sealing surface 221. In this case, an area of the sealing fit between the mounting cover 210 and the window 220 can be increased through the sealing fit between the third sealing surface 2121b and the fourth sealing surface 221, so as to improve the sealing performance of the window assembly 200 itself, thereby further improving the sealing performance of the endoscope.

In the embodiments of the present application, the window 220 may further include a retaining ring and a window glass. The window glass is fixedly mounted in the retaining ring, and the window glass is sealingly connected with the retaining ring. In order to improve the overall performance of the endoscope, the window glass may be made of sapphire, which has better optical and mechanical properties and lower power consumption. However, the window glass may be made of other materials which can better realize the function, which is not particularly limited in the present application.

In a further embodiment, in order to improve the assemblability of the mounting cover 210, the mounting cover 210 may further include a cover body section 212. The cover body section 212 and the sleeving section 211 are opposite to each other in an extension direction of the probe tube 100 and are connected to each other, such that the cover body section 212 is disposed at an end of the mounting cover 210 away from the probe tube 100. The cover body section 212 may include an annular mounting portion 2121, in which the mounting hole 2121a is provided. The annular mounting portion 2121 protrudes from an inner wall surface of the sleeving section 211, and an end surface of the opening end 111 is in contact with the annular mounting portion 2121 to limit the opening end 111. In this case, since an inner diameter of the sleeving section 211 is close to an outer diameter of the opening end 111 of the first tube body 110, an inner diameter of the annular hole of the annular mounting portion 2121 protruding from the inner wall surface of the sleeving section 211 is smaller than the outer diameter of the opening end 111 of the first tube body 110. Therefore, the annular mounting portion 2121 can play a stopping role when the mounting cover 210 is mounted on the first tube body 110, so as to avoid excessive movement of the mounting cover 210 during assembly on the first tube body 110.

In a more particular embodiment, the cover body section 212 and the sleeving section 211 may be of a one-piece structure, which is convenient to manufacture while reducing the assembly operations by operators, and thus can improve the economic performance of the endoscope.

In the endoscope according to the embodiments of the present application, the first sealing surface 2111 may be parallel to the second sealing surface 1111. In this case, the first sealing surface 2111 and the second sealing surface 1111, which are parallel to each other, are conducive to achieving a sleeving assembly therebetween, and have a uniform spacing distance therebetween, which is conducive to achieving the sealing fit, and further improving the sealing performance between the window assembly 200 and the probe tube 100.

In the endoscope according to the embodiments of the present application, the probe tube 100 may further include a second tube body 120. The second tube body 120 is disposed around the first tube body 110, and the second tube body 120 is disposed around the sleeving section 211. In this case, the second tube body 120 can protect the first tube body 110, and since the second tube body 120 is disposed around the sleeving section 211, a longer assembly path can be formed through the fit between the second tube body 120 and the sleeving section 211, which is helpful to improve the sealing performance between the first tube body 110 and the window assembly 200, that is, between the opening end 111 and the sleeving section 211.

In a further embodiment, an inner wall surface of the second tube body 120 may be spaced apart from an outer wall surface of the first tube body 110 to form a gap 130. The sleeving section 211 blocks an end of the gap 130 adjacent to the opening, and the inner wall surface of the second tube body 120, the outer wall surface of the first tube body 110, and the sleeving section 211 define an accommodation space for accommodating an optical fiber bundle. In this case, the optical fiber bundle can be disposed between the first tube body 110 and the second tube body 120 to provide a light source for the window assembly 200 of the endoscope, so as to enable the normal operation of the endoscope during observation, which is beneficial for the medical staff to view the site of lesion of the patient, to improve the practicability of the endoscope, and also to improve the accuracy of diagnosis and treatment by the medical staff.

In a more particular embodiment, the inner wall surface of the second tube body 120 may have a fifth sealing surface 121 opposite to the sleeving section 211, the sleeving section 211 has a sixth sealing surface 213, and the fifth sealing surface 121 is in sealing fit with the sixth sealing surface 213. In this case, the second tube body 120 may be merely disposed around the sleeving section 211, such that the inner wall surface of the second tube body 120 is in sealing fit with the sleeving section 211 through the fifth sealing surface 121 and the sixth sealing surface 213, that is, the sleeving section 211 is in sealing connection with both the first tube body 110 and the second tube body 120. Therefore, the sealing performance between the sleeving section 211 and the opening end 111 can be more effectively improved. However, the second tube body 120 may be disposed around the whole window assembly 200, for example, the second tube body 120 may be disposed around the mounting cover 210 so as to be disposed around the whole window assembly 200, such that the first tube body 110, the second tube body 120, and the mounting cover 210 can be sealingly connected with each other to form a sealed accommodation space, which is beneficial to protecting the structure disposed in the accommodation space.

In an optional embodiment, the gap 130 includes a first sub-gap 131 and a second sub-gap 132. The first sub-gap 131 may be formed between the first tube body 110 and the second tube body 120 on a first side of the first tube body 110. The second sub-gap 132 may be formed between the first tube body 110 and the second tube body 120 on a second side of the first tube body 110. The first side and the second side are opposite sides of the first tube body 110. A width of the first sub-gap 131 may be smaller than that of the second sub-gap 132, and the second sub-gap 132 is used to accommodate the optical fiber bundle. In this case, the first tube body 110 may be eccentrically disposed in the second tube body 120, such that the second sub-gap 132 having a larger width can facilitate the placement of objects, thereby facilitating the arrangement of the optical fiber bundle in the second sub-gap 132. The second sub-gap 132 having a larger width also facilitates providing a sufficient number of optical fiber bundles in the endoscope for providing sufficient illumination to the endoscope.

In the endoscope according to the embodiments of the present application, an end surface of the second tube body 120 adjacent to the opening may be a first end surface, an end surface of the window assembly 200 away from the opening may be a second end surface, and the first end surface and the second end surface are coplanar with each other, and both are arranged obliquely with respect to a first direction, where the first direction is perpendicular to the extension direction of the probe tube 100. In this case, neither the first end surface nor the second end surface is perpendicular to the extension direction of the probe tube 100, such that the first end surface and the second end surface can be coplanar with each other to form an inclined end surface in the endoscope, which can facilitate detection of the site of lesion during the use of the endoscope. Moreover, the first end surface and the second end surface arranged obliquely enable a wide visual range of the detection, making it more accessible to the medical staff.

It is to be noted that, as used herein, the terms "comprise," "include," or any other variant thereof, are intended to cover non-exclusive inclusion, such that a process, method, article, or apparatus that includes a list of elements includes not only those elements but also other elements that are not explicitly listed or that are inherent to such process, method, article, or apparatus. Without further limitation, an element defined by the phrase "including a..." does not preclude the presence of another identical element in the process, method, article, or apparatus including that element.

The embodiments of the present application have been described above with reference to the drawings, but the present application is not limited to the specific embodiments described above. The specific embodiments described above are merely illustrative and not limiting. Those ordinary skilled in the art may, under the inspiration of the present application, make many other forms without departing from the purpose of the present application and the scope of protection of the claims, all of which fall within the scope of protection of the present application.

## Claims

1. An endoscope, comprising a probe tube (100) and a window assembly (200), the probe tube (100) comprising a first tube body (110), the first tube body (110) comprising an opening end (111), and the window assembly (200) being in blocking fit with an opening at the opening end (111);
**characterized in that**
the window assembly (200) comprises a sleeving section (211) disposed around the opening end (111), an inner wall surface of the sleeving section (211) serves as a first sealing surface (2111), an outer wall surface of the opening end (111) serves as a second sealing surface (1111), and the first sealing surface (2111) is in sealing fit with the second sealing surface (1111).

2. The endoscope according to claim 1, wherein the window assembly (200) comprises a mounting cover (210) and a window (220),
the mounting cover (210) is provided with a mounting hole (2121a), and an inner wall surface of the mounting hole (2121a) serves as a third sealing surface (2121b),
at least a portion of the window (220) is secured in the mounting hole (2121a), and a surface of the window (220) facing the third sealing surface (2121b) serves as a fourth sealing surface (221), and
the third sealing surface (2121b) is in sealing fit with the fourth sealing surface (221).

3. The endoscope according to claim 2, wherein the mounting cover (210) further comprises a cover body section (212), the cover body section (212) and the sleeving section (211) being opposite to each other in an extension direction of the probe tube (100) and connected to each other,
wherein the cover body section (212) comprises an annular mounting portion (2121) in which the mounting hole (2121a) is provided, the annular mounting portion (2121) protrudes from the inner wall surface of the sleeving section (211), and an end surface of the opening end (111) is in contact with the annular mounting portion (2121) to limit the opening end (111).

4. The endoscope according to claim 3, wherein the cover body section (212) and the sleeving section (211) are of a one-piece structure.

5. The endoscope according to any one of claims 1 to 4, wherein the first sealing surface (2111) is parallel to the second sealing surface (1111).

6. The endoscope according to any one of claims 1 to 5, wherein the probe tube (100) further comprises a second tube body (120), the second tube body (120) is disposed around the first tube body (110), and the second tube body (120) is disposed around the sleeving section (211).

7. The endoscope according to claim 6, wherein an inner wall surface of the second tube body (120) is spaced apart from an outer wall surface of the first tube body (110) to form a gap (130), and the sleeving section (211) blocks an end of the gap (130) adjacent to the opening, and
the inner wall surface of the second tube body (120), the outer wall surface of the first tube body (110), and the sleeving section (211) define an accommodation space for accommodating an optical fiber bundle.

8. The endoscope according to claim 7, wherein the inner wall surface of the second tube body (120) is provided with a fifth sealing surface (121) opposite to the sleeving section (211), the sleeving section (211) is provided with a sixth sealing surface (213), and the fifth sealing surface (121) is in sealing fit with the sixth sealing surface (213).

9. The endoscope according to claim 7 or 8, wherein the gap (130) comprises a first sub-gap (131) and a second sub-gap (132), the first sub-gap (131) being formed between the first tube body (110) and the second tube body (120) on a first side of the first tube body (110), and the second sub-gap (132) being formed between the first tube body (110) and the second tube body (120) on a second side of the first tube body (110), wherein the first side and the second side are opposite sides of the first tube body (110), a width of the first sub-gap (131) is smaller than that of the second sub-gap (132), and the second sub-gap (132) is used for accommodating the optical fiber bundle.

10. The endoscope according to any one of claims 6 to 9, wherein an end surface of the second tube body (120) adjacent to the opening is a first end surface, an end surface of the window assembly (200) away from the opening is a second end surface, the first end surface and the second end surface are coplanar with each other, and both are arranged obliquely with respect to a first direction, wherein the first direction is perpendicular to an extension direction of the probe tube (100).
